# EUROPEAN PATENT SPECIFICATION

(11) **EP 1 789 800 B1**
(45) Date of publication and mention of the grant of the patent: **20.05.2009**
(21) Application number: 05785315.2
(22) Date of filing: 10.08.2005
(51) Int. Cl.: G01N 33/53, G01N 33/544, C12P 19/04, A61K 39/02

(54) **ILEITIS DIAGNOSTIC ASSAY**
ILEITIS-DIAGNOSETEST
DOSAGES DIAGNOSTIQUES POUR LUTTER CONTRE L'ILEITE

(30) Priority: 13.08.2004 US 918006
(43) Date of publication of application: 30.05.2007
(73) Proprietor: BOEHRINGER INGELHEIM VETMEDICA, INC., St. Joseph MO 64506-2002 (US)
(72) Inventor: KROLL, Jeremy, J., Urbandale, IA 50323 (US); ROOF, Michael, B., Ames, IA 50014 (US); EICHMEYER, Marc, A., Bondurant, IA 50035 (US)
(74) Representative: Hammann, Heinz
(86) International application number: PCT/US2005/028464
(87) International publication number: WO 2006/020730

(56) References cited:
- WO-A-02/26250
- KIM J ET AL: "IMMUNOHISTOCHEMISTRY AND POLYMERASE CHAIN REACTION FOR THE DETECTION OF LAWSONIA INTRACELLULARIS IN PORCINE INTESTINAL TISSUES WITH PROLIFERATIVE ENTEROPATHY" JOURNAL OF VETERINARY MEDICAL SCIENCE - NIHON JUIGAKU ZASSHI, JAPANESE SOCIETY OF VETERINARY SCIENCE, TOKYO, JP, vol. 7, no. 62, July 2000 (2000-07), pages 771-773, XP008006411 ISSN: 0916-7250
- WATARAI MASAHISA ET AL: "Enzyme-linked immunosorbent assay to detect Lawsonia intracellularis in rabbits with proliferative enteropathy." THE JOURNAL OF VETERINARY MEDICAL SCIENCE / THE JAPANESE SOCIETY OF VETERINARY SCIENCE JUN 2004, vol. 66, no. 6, June 2004 (2004-06), pages 735-737, XP002444742 ISSN: 0916-7250
- HOLYOAKE ET AL.: 'ENZYME-LINKED IMMUNOSORBENT ASSAY FOR MEASURING ILEAL SYMBIONT INTRACELLULARIS-SPECIFIC IMMUNOGLOBULIN G RESPONSE IN SERA OF PIGS' JOURNAL OF CLINICAL MICROBIOLOGY vol. 32, no. 8, August 1994, pages 1980 - 1985, XP009008617
- KROLL ET AL.: ' Lipopolysaccharide-Based Enzyme-Linked Immunosorbent Assay for Experimental Use in Detection of Antibodies to Lawsonia intracellularis in Pigs' CLINICAL AND DIAGNOSTIC LABORATORY IMMUNOLOGY vol. 12, no. 6, June 2005, pages 693 - 699, XP003002419
- BOESEN ET AL.: 'Development, characterization and diagnostic application of a monoclonal antibody specific for a proteinase K resistant Lawsonia intracellularis antigen' VETERINARY MICROBIOLOGY vol. 105, 2005, pages 199 - 206, XP004740818

## Description

### BACKGROUND OF THE INVENTION

### Field of the Invention

The present invention is broadly concerned with improved immunoassays for the detection of antibodies against *Lawsonia intracellularis*, as well as an effective antigen comprising and preferably consisting essentially of an antigenic extract of *L. intracellularis* lipopolysaccharide. The preferred assay is an indirect-type ELISA assay having excellent specificity and sensitivity, allowing use of the assay for detection of low levels of antibody in animal-derived specimens during initial stages of infection prior to the onset of clinical signs of disease.

### Description of the Prior Art

Porcine proliferative enteritis (PPE), known as ileitis, intestinal adenomatosis, hemorrhagic enteropathy, or necrotic enteritis, is a naturally occurring disease that can affect pigs from waning to young adult stage. PPE was formerly believed to be caused by a *camplyobacter*-like organist or ileal symbiont intracellularis. More recently, it has been established that the causative agent is *Lawsonia intracellularis*, an obligate intracellular, gram-negative bacterium. The disease is of economic importance owing to death loss, increased medication costs, poor weight gain and decreased food conversion in affected animals.

A key element in the rational therapy and effective control of PPE is a rapid and accurate identification of etiologic agents, PPE may be diagnosed by observation of gross lesions and is confirmed by observation of specific hystopathological lesions in which the intracellular curved rods are demonstrated by special staining methods incorporating the use of an anti-*Lawsonia* monoclonal antibody. Ideally, a final determination should be made through the isolation of the causative agent. However, the isolation and culture of *L. intracellularis* requires specialized cell culture techniques.

Attempts have been made in the past to develop rapid assays for the detection of anti-*Lawsonia* antibodies. Current serological-based assays such as indirect fluorescence antibody test (IFAT) and immuno-peroxidase assay (IPMA) demonstrate good sensitivity and specificity in the detection of anti*-Lawsonia* antibodies antemortem in pig serum. Knittel JP, Jordan DM, Schwartz KJ, et al. Evaluation of Antemorten Polymerase Chain Reaction and Serological Methods for Detection of Lawsonia Intracellularis-exposed Pigs. American Journal of Veterinarian Research, 59:722-726 (1998). Guedes RMC, Gebhart CJ, Deen J, et al, Validation of an Immunopreoxidase Monoloyer Assay as a Serological Test for Porcine Proliferative Enteropathy. Journal of Veterinarian Diagnostic Investigation. 14:528-530 (2002). However, neither of these assays is sensitive enough to detect the lower concentrations of anti-*Lawsonia* antibodies often found in pig serum during the initial exposure and onset of infection time periods. In addition, these prior assays rely on highly skilled technicians to accurately conduct the tests and interpret the results, e.g., the results are subjectively obtained by spending many hours looking into a microscope, analyzing wells illuminated by UV or natural light, searching for *L. intracellularis* or *L*. *intracellularis*-infected cells stained fluorescent green or red representing a "positive" sample.

Enzyme-linked immunoassays (ELISA) have also been developed for detection of anti-*Lawsonia* antibodies. These prior efforts failed to produce a sufficiently sensitive and specific assay owing to various limitations including poor antigen quality and quantity, variability in antibody titers, overlapping antibody titer between infected and non-infected pigs lack of a valid positive/negative "cut-off" value, and cross-reactivity of pig antibodies to non-specific antigen components. Holyoake PK, Cutler RS, Caple IW, Monckton RP. Enzyme-linked Immunosorbent Assay for Measuring Ileal Symbiont Intracellularis-specific Immunoglobulin G Response in Sera in Pigs. Journal of Clinical Microbiology. 31: 1980-1985 (1994).

WO 02/26250 provides a of *Lawsonia intracellularis* as well as monoclonal antibodies which specifically react with some of the antigens. The antigen have been identified by SDS-page electrophoresis and Western blotting of *Lawsonia intracellularis* antigen extracts with serum from convalescent pigs. It is described that the antigens as well as the monoclonal antibodies can be used for therapeutic and/or diagnostic purposes, However, WO 02/26250 does not provide an Lipopolysaccharides (LPS) preparations.

There is accordingly a need in the art for an improved anti-*Lawsonia* antibody assay which is highly sensitive and specific, permitting accurate detection of low concentrations of antibodies in animal-derived specimens during the early stages of infection.

Lipopolysaccharides (LPS) are a major suprastructure of gram-negative bacteria such as *L. intracellularis* which contributes greatly to the structural integrity of the bacteria, and protects them from host immune defenses. LPS's form a part of the Gram negative bacteria cell wall and comprise three parts: polysaccharide side chains, core polysaccharides and lipid A. Lipid A may contain unusual fatty acids (e.g., hydroxy-mysteric acids) while core polysaccharides often contain unusual sugars (e.g., KDO, keto-deoxyoctulonate and heptulose), The polysaccharide side chain is referred to as the O-antigen of the bacteria. LPS's function as endotoxin, because they can bind to the CD14 receptor of macrophage, triggering the whole cascade for macrophage/endothelial cells to secrete pro-inflammatory cytokines.

### SUMMARY OF THE INVENTION

The present invention overcomes the problems outlined above and provides an improved immunoassay for detecting the presence of antibodies against *Lawsonia intracellularis* with a high degree of specificity and sensitivity, allowing the assay to be used for the early detection of PPE. Broadly speaking, the assay of the invention is an immunoassay wherein an animal-derived specimen is contacted with an effective amount of an antigen comprising at least a portion of a lipopolysaccharide of *L. intracellularis*, causing the formation of complexes between the antigen and the antibodies, and then determining the presence of such antigen-antibody complexes. In preferred forms, the assay is an indirect ELISA test.

The animal-derived specimen is most preferably sera, but can also include colostrums, joint fluids, salivas, tissue homogenates and feces. These specimens can be prepared in accordance with conventional techniques for assay purpose. Although the invention is particularly concerned with detection of PPE in swine, analogous *Lawsonia*-caused diseases can also be detected in animals such as pigs, hamsters, blue fox, emus, deer, dogs, guinea pigs, horses, rhesus macaque monkeys, ostriches, rabbits and rats. The antibodies detected using the methods of the invention generally are selected from the group consisting of IgG, IgA and IgM antibodies.

The preferred antigen for use in the immunoassays of the invention is a portion or extract of a lipopolysaccharide of *L. intracellularis*. This extract preferably has a molecular weight of from about 15-25 kDa, more preferably from about 18-20 kDa. The extract or portions thereof should be of sufficient size and antigenicity to induce an immune response in the animal upon administration of an effective amount of the antigen. Furthermore, the extract or portion thereof must be of sufficient size for antibody-antigen complexes with LPS antibodies. In particularly preferred forms, the LPS antigen exhibits an endotoxicity of from about 3-75 EU/ml (more preferably from about 10-60 EU/ml, still more preferably from about 20-50, and even more preferably from about 25-40 EU/ml) using the bacteria endotoxin test. The antigen preferably consists essentially of LPS extract, and good results have been obtained with an extract of *L. intracellularis* ATCC Accession No. PTA-4927.

The present application finds utility with various types of immunoassays including but not limited to ELISAs such as competitive or inhibition assays and including direct and indirect assays, as well as sandwich or capture antibody assays. The most preferred immunoassay is an indirect-type ELISA, which involves first coating the wells of a microtiter plate with LPS, followed by overnight incubation at room temperature. A blocking agent is then added with further overnight incubation at 4EC. The selected dilute detection antibody is then added with incubation at 37EC for one hour, followed by diluted conjugate with further one hour (37EC) incubation. Next, the TMB chromagen is added with incubation at room temperature for five minutes. At this point the reaction is stopped with the addition of 2M sulfuric acid and the plate is read at 450 nm.

The preferred ELISA test is optimized in the case of a starting LPS extract sample of ATCC Accession No. PTA-4927 having an endotoxin level of about 34.75 EU/ml. In such an instance, the antigen should be present at a dilution of from about 1:250 to 1:8000, more preferably from about 1:400 to 1:6000, still more preferably from about 1:500 to 1:4000, even more preferably from about 1:600 to 1:3000, still more preferably from about 1:750 to 1:2000, even more preferably from about 1:900 to 1:1500, with 1:1,000 being optimum. The detection antibody should be present at a dilution level of about 1:20-1:320, 1:25-1:240, 1:30-1:128, 1:35-1:60 with 1:40 being the best The ELISA conjugate should be used at a level of from about 1:250-1:2000, 1:300-1:1500, 1:350-1:1000, 1:400-1:600, with 1:500 being preferred. Of course, appropriate dilution levels may be readily calculated if the starting antigen has a different endotoxin level than the preferred product of the invention.

The present invention also lends itself to recombinant LPS antigens being used as the antigenic source. In such instances, the LPS antigen or portion thereof will be generated using conventional recombinant techniques. For example, DNA encoding for the LPS antigen or desired portion thereof can be inserted into expression vectors and operably linked to expression control sequences which then permit the expression vector to express the desired LPS antigen or portion thereof. The expressed antigen is then recovered and used in accordance with the present invention. Of course, those of skill in the art are familiar with various ways of producing and recovering recombinant antigens in accordance with the present invention. In all instances, recombinant antigens in accordance with the present invention will preferentially bind to or hybridize with *L. intracellutaris* antibodies.

### DESCRIPTION OF THE PREFERRED EMBODIMENTS

The following examples set forth presently preferred techniques for constructing and using *Lawsonia* LPS as an antigenic source for immunoassays. Such examples include an indirect-type ELISA immunoassay against *Lawsonia* antibodies. It is to be understood, however, that such examples are provided by a way of illustration only, and nothing therein should be construed as a limitation upon the overall scope of the invention.

### EXAMPLE I

Development and Confirmation of LPS-based Indirect ELISA Assay

### Bacterial antigen preparation

The bacterial isolate was identified as high passage (> 20 passages past initial isolation from the affected gut) *L. intracellularis* isolate #15540 (ATTC Accession No. PTA-4927). This isolate was acquired from a Danish sow affected with acute hemorrhagic proliferative enteropathy (HPE), as confirmed by routine histology and immunohistochemistry (IHC) staining techniques and co-cultured to obtain a pure culture of *L. intracellularis* by methods previously described. Lawson GHK, McOrist S, Jasni , et al. Intracellular Bacteria of Porcine Proliferative Enteropathy: Cultivation and Maintenance in vitro. Journal of Clinical Microbiology. 31:1136-1142 (1993). Multiple 30 L batches of *L. intracellularis* #15540 were propagated using fresh McCoy cell (ATCC #1696) suspensions in bioreactors (Applicon, Inc., Foster City, CA), Active cultures were allowed to reach 80 - 100% cell infectivity and then harvested by centrifugation using an Avanti Beckman J-20I centrifuge (Beckman Instruments, Inc., Fullerton, CA), JA-10 rotor at 17,000 x g for 15 minutes at 4°C. The supernatants of each batch were discarded and cell pellets containing both extracellular *L. intracellularis* and McCoy cells infected with *L. intracellularis* were resuspended in 30 ml sterile 1X phosphate-buffered saline (PBS) and stored at -80°C.

In order to purify *L. intracellularis* from McCoy cells, a discontinuous percoll gradient was prepared following methods previously described with slight modifications. Holyoake PK, Cutler RS, Caple IW, Monckton RP. Enzyme-linked Immunosorbent Assay for Measuring Ileat Symbiont Intracellularis-specific Immunoglobulin G Response in Sera in Pigs. Journal of Clinical Microbiology. 31:1980-1985 (1994). Briefly, 225 ml of percoll (Amersham Biosciences, Pharmacia Biotech, Uppsala, Sweden) was mixed with 260 ml of reagent grade (RG) water and 15 ml of 5M sodium chloride (NaCl, Fisher Brand). Harvested cultures mentioned above were passed > 20 times through a 25 gauge needle and 5 ml of bacterial/McCoy cell homogenate was transferred to 25 ml of the percoll gradient into 30 ml polycarbonate centrifuge tubes. Tubes were mixed by inversion and centrifuged at 37,000 x g for 1 hour at 4°C. The ensuing suspension contained scattered cellular debris in the upper 50% of the tube while one distinct cellular banding pattern was visualized at a buoyant density of 1.075 g/ml. The upper half of the gradients were removed while the bands were carefully collected using a 5 ml polypropylene pipette and transferred to new 30 ml centrifuge tubes containing 20 ml of sterile PBS. The tubes were centrifuged (Avanti Beckman J-20I, JA-17 rotor) at 37,000 x g for 15 minutes at 4°C and the process repeated a maximum of 3 times to wash out the percoll from each sample. After the final centrifugation step, the pellets were resuspended in I to 2 ml of sterile PBS, pooled, aliquoted into 1.8 ml cryovial tubes (Nalgene, Nalgene Nune Int'l., Rochester, NY) and stored at -80°C. A sample of the resuspension containing highly concentrated, percoll purified *L. intracellularis* #15540 was observed under dark field microscopy to confirm presence of tiny curved rods and absence of intact McCoy cells. The LPS antigenic component was extracted from the percoll purified *L. intracellularis* #15540 antigen with hot aqueous phenol using methods previously described with slight modifications. Westphal, O. and Luderitz, O. Chemische Erforschung von Lipopolysacchariden Gramnegativer Bacterien. Angew. Chemical 66: 407-17 (1954). Briefly, 18 ml of percoll purified *L. intracellularis* and 3.75 ml of phenol chloroform (Ameresco, Solon, OH), pH 8.0 was incubated separately in a 65°C water bath for 10 minutes. After the initial incubation period, 4.5 ml of the percoll purified *L. intracellularis* was transferred to each tube containing 4.5 ml of a 90% (vol/vol) hot aqueous phenol suspension chloroform and gently mixed by inversion. Tubes were incubated for an additional 25 minutes in the 65°C water bath, mixing by inversion every 5 minutes during incubation and then cooled overnight at 4°C, Slight phase separation of aqueous and solid phases occurred in each tube during cold storage. Each tube was centrifuge (Avanti Beckman J-20I, JA-17 rotor) at 7,700 x g for 25 minutes at 4°C and the LPS-containing supernatant from all 4 tubes was pooled and retained while the cell pellets were discarded. The supernatant was transferred to pre-sterilized dialysis tubing (Spectrum Laboratories, Inc., Rancho Domingo, CA), placed in a 4L plastic beaker and dialyzed in cold reagent grade (reverse osmosis) water for 24 to 48 hours to remove the phenol chloroform from the sample. The reagent grade water was exchanged with fresh water every 2 to 4 hours until the washing step was complete. The resultant purified *L. intracellularis* LPS extract was carefully collected and stored at -80°C until use.

Confirmation of the LPS extract was visualized through separation on 4-12 % Bis-Tris pre-cast gel (NuPAGE, Invitrogen, Carlsbad, CA) by sodium dodecyl sulfate polyacrylamide gel electrophoresis (SDS-PAGE) in MOPS running buffer (NuPAGE, Invitrogen). The LPS extract was compared to percoll purified whole-cell *L. intracellularis* and uninfected whole-cell McCoy cells. Samples were prepared for SDS-PAGE by diluting each 1:2 into 4X lithium salt dodecyl sulfate (LDS) denaturing buffer (NuPAGE, Invitrogen) and incubated in a 85°C water bath for 10 minutes. The gel was periodate silver stained following procedures outlined by Bio-Rad Silver Stain instructions with modifications. In brief, the gel was subjected to a primary fixation in 40% Methanol (MeOH, Fisher Brand, Hanover Park, IL)/10% acetic acid (Fisher Brand) (v/v) for 20 to 30 minutes followed by incubation in 40% MeOH/10% acetic acid (v/v) containing 0.7% periodic acid for an additional 5 minutes at room temperature. A secondary fixation involved transferring the gel into 10% ethanol (EtOH)/5% acetic acid (v/v) for 5 minutes followed by oxidation in oxidizer reagent (Bio-Rad, Hercules, CA) for 5 minutes then, washed in deionized water until the yellow color was gone from the gel. The gel was incubated in 0.16mM dithiothreitol (DTT) in water for 5 minutes and stained in silver reagent (Bio-Rad) for 20 minutes at Room temperature. The gel was washed once in deionized water for 20 to 40 seconds and placed in developing reagent (Bio-Rad) until the desired strength of banding appeared. The reaction was stopped in 5% (v/v) acetic acid for 20 minutes at room temperature and washed once in deionized water. Proteins were identified by their molecular mass using a 10 to 220 kDa protein marker (BenchMark, Invitrogen).

### LPS-based Indirect ELISA

Anti-Lawsonia immunoglobulin G (IgG) antibodies were detected in test samples using an *L. intracellularis* LPS-based indirect enzyme linked immunosorbent assay (LPS-ELISA). *Lawsonia intracellularis* LPS was coated at 100 :1/well on Immulon 2HB plates (Dynex, Chantilly, VA) at a 1:1000 dilution in 0.05M sodium carbonate coating buffer, pH 9.6, sealed with mylar plate sealers (Thermo Labsystems, Franklin, MA) and incubated at room temperature for 24 hours. Each plate was washed using the Ultrawash PLUS (Dynex), 350 ml/well, zero soak time, for 1 wash cycle with wash buffer containing 0.05% Tween 20 (Fisher Brand), 0.137M NaCl (Fisher Brand), 0.005M potassium chloride (KCl, Sigma, St. Louis, MO), 0.009M Sodium Phosphate, diabasic (Na₂HPO₄, Sigma), 0.001M potassium phosphate (KH₂PO₄, Sigma), pH 7.2 to 7.4, in RG water, Antigen-coated plates were blocked at 300 ml/well with blocking buffer containing 5% (v/v) non-fat dried milk (Bio-Rad) in SeaBlock™ (Pierce Biotech, Rockford, IL, containing steelhead salmon serum and 0.1% sodium oxide in PBS) at 4°C for 24 hours to prevent non-specific binding of test sera to the plates. Each plate was then washed for 3 cycles as mentioned above. Test sera and controls were pre-diluted 1:40 in blocking buffer.

Fifty microliters per well of diluted sera was transferred to each plate in duplicate, sealed and incubated at 37°C for 1 hour. The washing steps were repeated for 3 cycles before adding 50 :l/well of a 1:500 dilution of goat anti-swine IgG horseradish peroxidase (HRP) conjugate (Kirkegaard and Perry Laboratories, Inc., Gaithersburg, MD) in blocking buffer. Plates were sealed and incubated at 37°C for 1 hour. The washing steps were repeated for 3 more cycles and then, 50 :l/well of peroxidase substrate consisting of a 2-component 3,3',5,5' tetramethylbenzidine (TMB, KPL) was applied to each plate and incubated at Room temperature for 5 minutes. This was done to observe the presence of antigen-antibody complexes within each test sample. The colorimetric reaction was stopped by transferring 50 :l/well of 2M sulfuric acid (H₂SO₄, Fisher Brand) solution. Plates were read at 450 nm wavelength on a V-max 96-well microtiter plate reader (Medtechs, Inc., Chapel Hill, NC) to obtain optical density (OD) values for each test sample. Standard controls containing a range of high positive to low positive (by serial two-fold dilution of the highly positive sample in blocking buffer) and negative antibodies against the *L*. *intracellularis* LPS extract were included in each plate, The test plates were considered valid if the coefficient of determination (r²-value) was ³0.9 in a linear regression analysis of the standard's OD values. An empty well not containing test or control samples served as a blank control for all tests.

### Validation of LPS-ELISA

Porcine sera were obtained from the following studies for use in validating the LPS-ELISA for detection of anti-*Lawsonia* IgG antibodies in pigs. (i) Two pigs approximately 3 weeks of age were hyperimmunized by intramuscular injection with 2 ml of *L. intracellularis* LPS extract formulated 1:2 with Freund's incomplete adjuvant (Sigma). One pig approximately 3. weeks of age was given a 2 ml intramuscular injection of placebo consisting of Dulbecco's Minimal Essential Medium (DMEM) with 5% (v/v) bovine serum (JRH Biosciences, Lenexa, KS) formulated 1:2 with Freund's incomplete adjutant Boosters of each inoculum were administered 3 weeks and 6 weeks post initial inoculation for generation of positive (antibodies to *L*. *intracellularis* LPS) and negative (no *L. intracellularis* antibodies) control test serum. Four to six milliliters of serum was collected from each pig prior to inoculation, at 3 weeks and 6 weeks post inoculation and tested for reaction of *L*. *intracellularis* whole cell antigen by an indirect florescent antibody serology test (IFAT), Knittel JP, Jordan DM, Schwartz JK, et al. Evaluation of Antemorten Polymerase Chain Reaction and Serologic Methods for Detection of Lawsonia Intracellularis-exposed Pigs. American Journal of Veterinarian Research. 59:722-726 (1998), to confirm presence or absence of anti-*Lawsonia* antibodies in serum samples. At 8 weeks post-initial inoculation, animals were euthanised and a final serum collection was obtained. The positive control serum from each pig was pooled, tested to confirm positive reaction to *L. intracellularis* by IFAT, and both anti-Lawsonia LPS positive and negative control serum was stored in 1 ml aliquots at -80°C.

Porcine sera from 2 previously conducted vaccine efficacy studies, Kroll, J., et al. (2004). Evaluation of protective immunity in pigs following oral administration of an avirulent live vaccine of Lawsonia intracellularis, AJVR 65(5): 559-565 were tested to investigate an anti-*Lawsonia* LPS antibody positive/negative optical density cut-off limit. Test serum from eighty 6 to 9 week old pigs previously confirmed to be IFAT-positive for anti-*Lawsonia* antibodies were generated after experimental infection with a virulent heterologous *L*. *intracellularis* isolate N101494 (Boehringer Ingelheim Vetmedica, Inc., St. Joseph, MO). Test serum previously confirmed to be IFAT-negative, was collected from eighty 3 to 9 week old strict control pigs that did not receive a vaccination or challenge any time during each clinical study.

175 serum samples were collected from 25 pigs after vaccination with a live attenuated *L. intracellularis* vaccine, Enterisol^{®} Ileitis (Boehringer Ingelheim Vetmedica, Inc.), after challenge with a virulent heterologous *L. intracellularis* isolate N101494 or both. Another 70 serum samples were collected from 10 strict control pigs that did not receive a vaccination or challenge and remained IFAT negative for anti-*Lawsonia* antibodies throughout the study. The study design included thirty five 3 to 4 week old pigs randomly blocked into 3 treatment groups. On day 0 of the study, 15 pigs from group 1 received a 2 ml oral dose of vaccine while groups 2 and 3 (10 pigs/group) received an equivalent dose of placebo consisting of uninfected McCoy cell suspension in growth medium. On day 21, pigs in groups 1 and 2 were given an intragastric dose of virulent heterologous pure culture challenge of *L. intracellularis* N101494. On day 42, pigs were necropsied and evaluated for lesion development to identify efficacy of the vaccinated pigs compared to non-vaccinated, challenged pigs. Fecal samples and serum were collected weekly from day 0 to 42 for routine diagnostic testing to detect rates of exposure and active shedding of *L. intracellularis* due to vaccine or challenge administration. Lesions were evaluated to confirm presence of PE at day 42 only by gross examination, histological and IHC methods as described below.

### Confirmation of PE in pigs

Gross lesions found in the ileum or colon of pigs in clinical studies described above were scored according to the severity of mucosal thickness (1 = normal, 2 = mild thickening, 3 = moderate thickening/inflammation, 4 = severe ihickening/inflammation/mucosal hemorrhaging or necrosis may be present). Kroll, J., et al. (2004). Evaluation of protective immunity in pigs following oral administration of an avirulent live vaccine of Lawsonia intracellularis. AJAR 65(5): 559-565. Samples 2 - 4 cm in length of ileum and colon were collected post mortem, fixed by immersion in buffered formalin and processed for detection of microscopic lesions. This included Hematoxylin and Eosin (H&E) and IHC staining incorporating specific *L. intracellularis* monoclonal antibodies. Kroll, J., et al., AJVR, (2004). The latter is considered the current standard for assessment of the actual infection status of a pig with *L. intracellularis*. Kroll, J., et al. (2004). Microscopic lesions found in IHC stained tissue were scored separately according to severity of *L. intracellularis* specific cell proliferation (0 = normal, 1 = mild/focal, 2 = moderate/diffuse, 3 = severe/diffuse). Average gross and microscopic lesion scores and the frequency of lesions detected in the affected tissue were calculated for group comparisons. Average gross and macroscopic lesion scores were considered the primary parameter for determining vaccine efficacy against virulent heterologous challenge in previous studies. Kroll, J., et al. (2004).

### EXAMPLE II

### Preferred LPS-ELISA Materials and Methods

The following sets forth the presently preferred LPS-ELISA assay in accordance with the invention.
**A. Protocol**
   **1. Materials**
      a. LPS binding Plates:
         Immunlon 2 HB 96 weal plates, Dynex Cat No. 3455 or equivalent.
      b. Dilution Plates:
         Falcon Pro-Bind Assay Plate (Fisher Scientific, Pittsburg, PA), 96-well, U-Bottom without lid (polystyrene, non-sterile), Becton Dickinson (San Diego, CA) Cat. No. 353910 or equivalent.
      c. Plate Sealers:
         Mylar Plate Sealer, Thermo Labsystems (Franklin, MA) Cat. No. 5701 or equivalent.
      d. Coating Buffer:
         0.05M Sodium Carbonate buffer
            10.6 g Na₂CO₃ Sigma Cat. No. S6139 or equivalent.
            QS with reagent grade (RG) H₂O (or equivalent) to 1.0 L.
            pH = 9.6 ± 0.1
            Store at 2 - 7 °C until use.
            Expiry: 7 days.
      e. Wash Solution:
         0.05% Tween 20, 0.137M NaCl, 0.005M KCl, 0.009M Na₂HPO₄, 0.001M KH₂PO₄
            32.0 g NaCl.
            0.8 g KCl.
            2.44 g Na₂HPO₄.
            0.8 g KH₂PO₄.
            QS with RG H₂O or equivalent to 4.0 L.
            pH to 7.2-7.4 with NaOH or HCl.
            2.0 ml of Tween, Fisher Cat No. BP337-100 or equivalent.
            Store at room temperature (25 V 5°C) until use.
            Expiry: 1 week.
      f. Blocking Solution:
         5% Non-fat dry milk in Seblock^{™}.
            25.0 g Non-fat dry mil. Bio-Rad Cat No. 170-6404 or equivalent.
            QS to 500 mL with Seablock^{™}. Pierce Biotech
            Cat. No. 37527 or equivalent.
            Store at 2 - 7 °C until use.
            Expiry: 1 moth.
      g. Antigen:
         1:1000 dilution of lipopolysaccaride whole molecules from *L. intracellularis*.
            40:1 *L*. *intracellularis* LPS into 40 ml of coating buffer.
            Use immediately.
      h. Detention Antibody:
         1:40 dilution of convalescent pig serum antibodies to *L*. *intracellularis.*
            3 :1 pig serum or equivalent into 120 :1 of blocking solution.
            Store at 2-7°C until use.
            Expiry: 24 hours.
      i. Conjugate antibody:
         1:500 dilution of Goat anti-mouse IgG (H+L) - Horse Radish Peroxidase (HRP). Kierkegaard and Perry Laboratories, Inc. Cat. No. 14-14-06 or equivalent.
            40 :1 conjugate into 20 ml of blocking solution.
            Store at 2-7°C until use.
            Expiry:24 hours.
      j. Substrate:
         Two-Component Microwell Peroxidase Substrate (Gaithersburg, MD). KPL Cat No. 50-76-00 or equivalent.
            Mix equal volumes of TMB Peroxidase Substrate (Reagent A) with Peroxidase Solution B (Reagent B) immediately prior to use.
            Volume required = 5 mL/plate. Therefore, 2.5 mL of Reagent A + 2.5 mL of Reagent B for 1 test plate.
            Store at 2-7°C until use.
            Expiry: Pre-mixed reagents per manufacturers's suggested expiration date. Use mixed substrate solution immediately.
      k. Stop Solution:
         2M H₂SO₄.
            In a fume hood, carefully mix: 444.4 mL of RG H₂0.
            55.6 mL of 18M H₂SO₄, Fisher Cat No. A300c-212, or equivalent.
            Store at room temperature until use.
            Expiry: 6 months.
      l. Positive Control
         1: 2,564 dilutions of hyperimmunized pig serum containing anti-*Lawsonia* LPS IgG antibodies.
            3.9:1 of positive control Lot #090203 into 10 ml of blocking solution
            Store at 2-7°C until use.
            Expiry: 24 hours
      m. Negative Control
         1: 2,564 dilution of hyperimmunized pig serum containing no antibodies against *L. intracellularis* LPS molecules.
            3.9 :1 of positive control Lot #090203 into 10 ml of blocking solution
            Store at 2-7°C until use.
            Expiry: 24 hours
   **2. Methods**
      a. Samples are run in duplicate. Number of plates needed = Total number of samples/40 samples per plate. Round up to a whole number of plates. Columns 11 and 12 will contain 1:10 serial dilutions of negative and positive control serum.
      b. Dilute *L. intracellularis* LPS antigen 1:1000 or appropriate working dilution in coating buffer. Volume required = Number of plates x 10 ml/plate.
      c. Add 100 ml of diluted antigen to every well of each plate.
      d. Seal plates with plate sealers and incubate at room temperature overnight (14-24 hours).
      e. Wash plates with wash solution using Dynex Ultrawash PLUS, 350 ml/well, zero soak time, for 1 wash cycle. Tap plates dry on paper towels.
      f. Add 300 ml of block solution to all wells. Seal plates and incubate at 2-7°C overnight (14-24 hours).
      g. Wash plates with wash solution using Dynex Ultrawash PLUS, 350 µl/well, zero soak time, for 3 wash cycles. Tap plates dry on paper towels.
      h. In a U-bottom dilution plate, add 120 µl of blocking solution sample to wells in columns 1 - 10 and wells B - H in columns 11 and 12.
      i. Add 240 µl of negative and positive controls in wells of row A in columns 11 and 12 respectively.
      j. Make 10-fold dilutions of each control serum by transferring 120 µl of diluted control in wells A-11 and A-12 to wells B-11 and B-12 using a 50 - 300 µl multi-channel pipette, taking care not to transfer diluted negative control to the last well of column 11 (well H-11) as this sample will serve as a plate black.
      k. Dilute detection antibody (convalescent pig sera) 1:40 in blocking solution by transferring 3 µl of sample into 120 µl of blocking solution in each well of the dilution plate. Dilute by adding a different sample each time to wells A-1, B-1, C-1, etc.
      l. Using a 50-300 µl multichannel pipette, mix the contents in column 1 by pipetting up and down at least 3 times and transfer 50 µl/ell to columns 1 and 2 of the antigen coated LPS-binding test plates. Change tips and repeat step until all diluted samples have been transferred in duplicate across the plate.
      m. Transfer 50 µl/well of the negative control (wells A - H, column 11) to corresponding wells of the test plate(s). Repeat step for the positive control. Each control contains enough diluted sample to use in 2 test plates.
      n. Seal test plate(s) with plate sealers and incubate for 1.0 hour ± 15 minutes at 37°C ± 2.0°C.
      o. Wash plates with wash solution using Dynex Ultrawash PLUS, 350 µl/well, zero soak time, for 3 wash cycles, Tap plates dry on paper towels.
      p. Add 50 µL of conjugate antibody diluted 1:500 or appropriate working dilution to all wells of the test plate(s). Volume required = Number of plates x 5 ml/plate.
      q. Seal test plate(s) with plate sealers and incubate for 1.0 hour ± 15 minutes at 37°C ± 2.0°C.
      r. Wash plates with wash solution using Dynex Ultrawash PLUS, 350 µl/well, zero soak time, for 3 wash cycles. Tap plates dry on paper towels.
      s. Add 50 µL of substrate, to use to all wells of the test plate(s), incubate at room temperature for five minutes ± 1 minute.
      t. Stop the reaction with the addition of 50 µl of Stop solution to all wells five minutes after the addition of substrate.
      u. Read plates on a plate reader equipped with a 450 nm wavelength filter.
   **3. Interpretation of Results**
      a. Test samples exhibiting > 0.200 optical density at 450nm wavelength aie considered positive for anti-*Lawsonia* LPS IgG antibodies.
      b. Test samples exhibiting < 0.200 optical density at 450nm wavelength are considered negative for anti-*Lawsonia* LPS IgG antibodies.

## Claims

1. A method for detecting the presence of antibodies against *Lawsonia intracellularis* in an animal-derived specimen in an immunoassay, comprising the steps of contacting said specimen with an effective amount of an antigen consisting essentially of a lipopolysaccharide extract of *Lawsonia intracellularis*, causing the formation of complexes between said antigen and said antibodies, and determining the presence of said complexes.

2. The method of claim 1, wherein said specimen selected from the group consisting of animal-derived sera, colostrums, joint fluids, salivas, tissue homogenates, and feces.

3. The method of claim 1, wherein said animal selected from the group consisting of pigs, hamsters, blue fox, emus, deer, dogs, guinea pigs, horses, rhesus macaque monkeys, ostriches, rabbits and rats.

4. The method of claim 1, wherein said antibodies being selected from the group consisting of IgG, IgA and IgM antibodies.

5. The method of claim 1, wherein said immunoassay being an ELISA test.

6. The method of claim 5, wherein said antigen being recombinantly deprived. .

7. The method of claim 5, wherein said ELISA test including the steps of immobilizing said antigen on a surface, contacting said specimen with said immobilized antigen, causing a complex between antibodies in said specimen and said antigen, and detecting said complexes.

8. The method of claim 1, wherein said lipopolysaccharide extract having a molecular weight of from 15-25 kDa.

9. The method of claim 8, wherein said molecular weight being from 18-20 kDa.

10. The method of claim 1, wherein said antigen capable of inducing an immune response in said animal upon administration of an effective amount of the antigen to the animal.

11. The method of claim 1, wherein said antigen exhibiting an endotoxicity of from 3-75 EU/ml using the bacterial endotoxin test.

12. The method of claim 11, wherein said endotoxicity being from 25-40 EU/ml.

13. The method of claim 1, wherein said antigen consisting essentially of a lipopolysaccharide extract of ATCC Accession No. PTA-4927.

14. An antigen for detecting the presence of antibodies against *Lawsonia intracellularis*, wherein said antigen consisting essentially of a lipopolysaccharide extract of *Lawsonia intracellularis*.

15. The antigen of claim 14, wherein said lipopolysaccharide extract having a molecular weight of from 15-25 kDa.

16. The antigen of claim 14, wherein said lipopolysaccharide extract having a molecular weight of from 18-20 kDa.

17. The antigen of claim 15, wherein said antigen capable of inducing an immune response in said animal upon administration of an effective amount of the antigen to the animal.

18. The antigen of claim 15, wherein said antigen exhibiting an endotoxicity of from 3-75 EU/ml using the bacterial endotoxin test.

19. The antigen of claim 15, wherein said lipopolysaccharide being recombinantly derived.

20. The antigen of claim 18, wherein said endotoxicity being from 25-40 EU/ml.

21. The antigen of claim 14, wherein said antigen consisting essentially of a lipopolysaccharide extract of ATCC Accession No. PTA-4927.

22. An enzyme-linked immunoassay for detecting the presence of antibodies against *Lawsonia intracellularis* in an animal-derived specimen comprising antigen, wherein said antigen consisting essentially of a lipopolysaccharide extract of *Lawsonia intracellularis*.

23. The enzyme-linked immunoassay of claim 22, wherein said animal-derived specimen is selected from the group consisting of pigs, hamsters, blue fox, emus, deer, dogs, guinea pigs, horses, rhesus macaque monkeys, ostriches, rabbits and rats.

24. The enzyme-linked immunoassay of claim 22, wherein said antibodies being selected from the group consisting of IgG, IgA and IgM antibodies.

25. The enzyme-linked immunoassay of claim 22, said ELISA test being an indirect-ELISA test.

26. The enzyme-linked immunoassay of claim 22, wherein said lipopolysaccharide extract having a molecular weight of from 15-25 kDa.

27. The enzyme-linked immunoassay of claim 22, wherein said lipopolysaccharide extract having a molecular weight from 18-20 kDa.

28. The enzyme-linked immunoassay of claim 22, wherein said antigen exhibiting an endotoxicity of from 3-75 EU/ml using the bacterial endotoxin test.

29. The enzyme-linked immunoassay of claim 28, wherein said endotoxicity being from 25-40 EU/ml.

30. The enzyme-linked immunoassay of claim 22, wherein said antigen essentially consisting of a lipopolysaccharide extract of ATCC Accession No. PTA-4927.

## Patentansprüche

1. Verfahren zum Nachweisen des Vorliegens von Antikörpern gegen *Lawsonia intracellularis* in einer von einem Tier stammenden Probe in einem Immunoassay, umfassend die folgenden Schritte: Inkontaktbringen der Probe mit einer wirksamen Menge eines Antigens, das im Wesentlichen aus einem Lipopolysaccharidextrakt von *Lawsonia intracellularis* besteht, Bildung von Komplexen zwischen dem Antigen und den Antikörpern und Bestimmung des Vorliegens dieser Komplexe.

2. Verfahren nach Anspruch 1, wobei die Probe aus der Gruppe bestehend aus von einem Tier stammenden Serum, Colostrum, Gelenkflüssigkeit, Speichel, Gewebehomogenisaten und Faeces ausgewählt ist.

3. Verfahren nach Anspruch 1, wobei das Tier aus der Gruppe bestehend aus Schweinen, Hamstern, Blaufüchsen, Emus, Hirschen, Hunden, Meerschweinchen, Pferden, Rhesus-Makaken, Straußen, Kaninchen und Ratten ausgewählt ist.

4. Verfahren nach Anspruch 1, wobei die Antikörper aus der Gruppe bestehend aus IgG-, IgA- und IgM-Antikörpern ausgewählt sind.

5. Verfahren nach Anspruch 1, wobei es sich bei dem Immunoassay um einen ELISA-Test handelt.

6. Verfahren nach Anspruch 5, wobei das Antigen auf rekombinante Weise erhalten worden ist.

7. Verfahren nach Anspruch 5, wobei der ELISA-Test folgende Schritte umfasst: Immobiliseren des Antigens auf einer Oberfläche, Inkontaktbringen der Probe mit dem immobilisierten Antigen in Kontakt, Bildung eines Komplexes zwischen Antikörpern in der Probe und dem Antigen und Nachweisen der Komplexe.

8. Verfahren nach Anspruch 1, wobei der Lipopolysaccharidextrakt ein Molekulargewicht von 15-25 kDa aufweist.

9. Verfahren nach Anspruch 8, wobei das Molekulargewicht 18-20 kDa beträgt.

10. Verfahren nach Anspruch 1, wobei das Antigen dazu befähigt ist, eine Immunantwort in dem Tier bei Verabreichung einer wirksamen Menge des Antigens an das Tier zu induzieren.

11. Verfahren nach Anspruch 1, wobei das Antigen eine Endotoxizität von 3-75 EU/ml bei Anwendung des bakteriellen Endotoxin-Tests aufweist.

12. Verfahren nach Anspruch 11, wobei die Endotoxizität 25-40 EU/ml beträgt.

13. Verfahren nach Anspruch 1, wobei das Antigen im Wesentlichen aus einem Lipopolysaccharidextrakt von ATCC Hinterlegungsnummer PTA-4927 besteht.

14. Antigen zum Nachweisen des Vorliegens von Antikörpern gegen *Lawsonia intracellulatis*, wobei das Antigen im Wesentlichen aus einem Lipopolysaccharidextrakt von *Lawsonia intracellularis* besteht.

15. Antigen nach Anspruch 14, wobei der Lipopolysaccharidextrakt ein Molekulargewicht von 15-25 kDa aufweist.

16. Antigen nach Anspruch 14, wobei der Lipopolysaccharidextrakt ein Molekulargewicht von 18-20 kDa aufweist.

17. Antigen nach Anspruch 15, wobei das Antigen dazu befähigt ist, eine Immunantwort bei einem Tier bei Verabreichung einer wirksamen Menge des Antigens an das Tier zu induzieren.

18. Antigen nach Anspruch 15, wobei das Antigen eine Endotoxizität von 3-75 EU/ml bei Anwendung des bakteriellen Endotoxin-Tests aufweist.

19. Antigen nach Anspruch 15, wobei das Lipopolysaccharid auf rekombinante Weise erhalten worden ist.

20. Antigen nach Anspruch 18, wobei die Endotoxizität 25-40 EU/ml beträgt.

21. Antigen nach Anspruch 14, wobei das Antigen im Wesentlichen aus einem Lipopolysaccharidextrakt von ATCC Hinterlegungsnummer PTA-4927 besteht.

22. ELISA-Test (enzyme-linked immunoassay) zum Nachweisen des Vorliegens von Antikörpern gegen *Lawsonia intracellularis* in einer von einem Tier stammenden Probe, umfassend Antigen, wobei das Antigen im Wesentlichen aus einem Lipopolysaccharidextrakt von *Lawsania intracellularis* besteht.

23. ELISA-Test nach Anspruch 22, wobei die von einem Tier stammende Probe aus der Gruppe bestehend aus Schweinen, Hamstern, Blaufüchsen, Emus, Hirschen, Hunden, Meerschweinchen, Pferden, Rhesus-Makaken, Straußen, Kaninchen und Ratten ausgewählt ist.

24. ELISA-Test nach Anspruch 22, wobei die Antikörper aus der Gruppe bestehend aus IgG-, IgA- und IgM-Antikörpern ausgewählt sind.

25. ELISA-Test nach Anspruch 22, wobei es sich um einen indirekten ELISA-Test handelt.

26. ELISA-Test nach Anspruch 22, wobei der Lipopolysaccharidextrakt ein Molekulargewicht von 15-25 kDa aufweist.

27. ELISA-Test nach Anspruch 22, wobei der Lipopolysaccharidextrakt ein Molekulargewicht von 18-20 kDa aufweist.

28. ELISA-Test nach Anspruch 22, wobei das Antigen eine Endotoxizität von 3-75 EU/ml bei Anwendung des bakteriellen Endotoxin-Tests aufweist.

29. ELISA-Test nach Anspruch 28, wobei die Endotoxizität 25-40 EU/ml beträgt.

30. ELISA-Test nach Anspruch 22, wobei das Antigen im Wesentlichen aus einem Lipopolysaccharidextrakt von ATCC Hinterlegungsnummer PTA-4927 besteht.

## Revendications

1. Procédé de détection de la présence d'anticorps dirigés contre *Lawsonia intracellularis* dans un échantillon provenant d'un animal dans le cadre d'un dosage immunologique, comprenant les étapes consistant à mettre en contact ledit échantillon avec une quantité efficace d'un antigène essentiellement constitué d'un extrait lipopolysaccharidique de *Lawsonia intracellularis*, ce qui provoque la formation de complexes entre ledit antigène et lesdits anticorps, puis à déterminer la présence desdits complexes.

2. Procédé selon la revendication 1, dans lequel ledit échantillon est choisi dans le groupe constitué des sérums, colostrums, liquides articulaires, salives, homogénats tissulaires et selles d'animaux.

3. Procédé selon la revendication 1, dans lequel ledit animal est choisi dans le groupe constitué des cochons, hamsters, renards bleus, émeus, cerfs, chiens, cochons d'Inde, chevaux, singes macaques rhésus, autruches, lapins et rats.

4. Procédé selon la revendication 1, dans lequel lesdits anticorps sont choisis dans le groupe constitué des anticorps de type IgG, IgA et IgM.

5. Procédé selon la revendication 1, dans lequel ledit dosage immunologique correspond à un test ELISA.

6. Procédé selon la revendication 5, dans lequel ledit antigène peut être obtenu par des techniques recombinantes.

7. Procédé selon la revendication 5, dans lequel ledit test ELISA comprend les étapes consistant à immobiliser ledit antigène sur une surface, à mettre en contact ledit échantillon avec ledit antigène immobilisé, ce qui provoque la formation d'un complexe entre les anticorps présents dans ledit échantillon et ledit antigène, et à détecter lesdits complexes.

8. Procédé selon la revendication 1, dans lequel la masse moléculaire dudit extrait lipopolysaccharidique se situe dans une fourchette de 15 à 25 kDa.

9. Procédé selon la revendication 8, dans lequel ladite masse moléculaire se situe dans une fourchette de 18 à 20 kDa.

10. Procédé selon la revendication 1, dans lequel ledit antigène se révèle capable d'induire une réponse immunitaire chez ledit animal suite à l'administration d'une quantité efficace de l'antigène à l'animal.

11. Procédé selon la revendication 1, dans lequel ledit antigène présente une endotoxicité de l'ordre de 3 à 75 UE/ml selon l'essai US, connu sous le nom de « bacterial endotoxin test ».

12. Procédé selon la revendication 11, dans lequel ladite endotoxicité varie de 25 à 40 UE/ml.

13. Procédé selon la revendication 1, dans lequel ledit antigène est essentiellement constitué d'un extrait lipopolysaccharidique dont le numéro d'accession ATCC est PTA-4927.

14. Antigène utilisable pour la détection d'anticorps dirigés contre *Lawsonia intracellularis*, ledit antigène étant essentiellement constitué d'un extrait lipopolysaccharidique de *Lawsonia intracellularis*.

15. Antigène selon la revendication 14, dans lequel ledit extrait lipopolysaccharidique présente une masse moléculaire de 15 à 25 kDa.

16. Antigène selon la revendication 14, dans lequel ledit extrait lipopolysaccharidique présente une masse moléculaire de 18 à 20 kDa.

17. Antigène selon la revendication 15, ledit antigène se révélant capable d'induire une réponse immunitaire chez ledit animal suite à l'administration d'une quantité efficace de l'antigène à l'animal.

18. Antigène selon la revendication 15, ledit antigène présentant une endotoxicité de 3 à 75 UE/ml selon l'essai US connu sous le nom de « bacterial endotoxin test ».

19. Antigène selon la revendication 15, dans lequel ledit lipopolysaccharide est obtenu par des techniques recombinantes.

20. Antigène selon la revendication 18, dont l'endotoxicité se situe dans une fourchette de 25 à 40 UE/ml.

21. Antigène selon la revendication 14, ledit antigène étant essentiellement constitué d'un extrait lipopolysaccharidique dont le numéro d'accession ATCC est PTA-4927.

22. Dosage par méthode immunoenzymologique permettant de détecter la présence d'anticorps dirigés contre *Lawsonia intracellularis* dans un échantillon provenant d'un animal comprenant un antigène, ledit antigène étant essentiellement constitué d'un extrait lipopolysaccharidique de *Lawsonia intracellularis*.

23. Dosage par méthode immunoenzymologique selon la revendication 22, dans lequel ledit échantillon provient d'un animal choisi dans le groupe constitué des cochons, hamsters, renards bleus, émeus, cerfs, chiens, cochons d'Inde, chevaux, singes macaques rhésus, autruches, lapins et rats.

24. Dosage par méthode immunoenzymologique selon la revendication 22, dans lequel lesdits anticorps sont choisis dans le groupe constitué des anticorps de type IgG, IgA et IgM.

25. Dosage par méthode immunoenzymologique selon la revendication 22, dans lequel ledit test ELISA correspond à un test ELISA indirect.

26. Dosage par méthode immunoenzymologique selon la revendication 22, dans lequel la masse moléculaire dudit extrait lipopolysaccharidique se situe dans une fourchette de 15 à 25 kDa.

27. Dosage par méthode immunoenzymologique selon la revendication 22, dans lequel la masse moléculaire dudit extrait lipopolysaccharidique se situe dans une fourchette de 18 à 20 kDa.

28. Dosage par méthode immunoenzymologique selon la revendication 22, dans lequel ledit antigène présente une endotoxicité de l'ordre de 3 à 75 UE/ml selon l'essai US connu sous le nom de « bacterial endotoxin test ».

29. Dosage par méthode immunoenzymologique selon la revendication 28, dans lequel ladite endotoxicité varie de 25 à 40 UE/ml.

30. Dosage par méthode immunoenzymologique selon la revendication 22, dans lequel ledit antigène est essentiellement constitué d'un extrait lipopolysaccharidique dont le numéro d'accession ATCC est PTA-4927.
